# EUROPEAN PATENT APPLICATION

(11) **EP 3 106 844 A1**
(43) Date of publication of application: **21.12.2016**
(21) Application number: 15172662.7
(22) Date of filing: 18.06.2015
(51) Int. Cl.: G01F 23/292, A61B 17/435, G01N 21/00

(54) **DROP AND LEVEL DETECTION OF HUMAN FOLLICLE FLUID IN A TEST TUBE**

(71) Applicant: Shivani Scientific Industries Private Limited, 401104 Mumbai (IN)
(72) Inventor: Kale, Ravikant, 401104 Mumbai (IN); Modi, Ashish, 401104 Mumbai (IN)
(74) Representative: Nordmeyer, Philipp Werner

(57) **Abstract**

Invention is an apparatus and a method for detecting first and subsequent drops of follicle fluid falling into a test tube as well as level of the follicle fluid falling into a test tube during an aspiration process. In one aspect of the Invention a pair of optical devices (emitter and detector) is positioned at opposite side of the test tube. The emitter emits a beam of light towards the detector. In another aspect the apparatus may be configured for detecting the level of the follicle fluid falling into the test tube. Upon detecting that the level reaches the pre-defined threshold level or droplet falling in the test tube, the apparatus may be further configured to notify a doctor via an audio alarm or visually through the ultrasound monitor.

## Description

### TECHNICAL FIELD

The present disclosure described herein, relates to a method and apparatus for detection of drop of a human follicle fluid as well as detection of level of follicle fluid falling into a test tube.

### BACKGROUND

'IVF' (In Vitro Fertilization) is one of the treatments for infertility in medical science. As part of the IVF procedure, it is required that an oocyte (egg) is extracted from a female patient in an operation theatre while the patient is under anesthesia. The oocyte comprises an egg that can be fertilized outside the female patient body with a sperm in order to produce an embryo. The embryo may then be transferred to the uterus of the patient to facilitate successful pregnancy.

In the present day situation, an Ultrasound Oocyte Recovery procedure is performed by a doctor for extracting oocyte from the ovaries of a female patient. In the said procedure, a vaginal ultrasound probe with an attached needle guide is inserted into the patient's vagina under sterile conditions and the needle is further passed through the top of the vagina into the ovary. The follicles in the ovary are then aspirated until the oocyte is obtained. An apparatus known as 'aspiration pump' or 'aspirator' is utilized for generating a sufficient amount of vacuum that enables the extraction of the follicle fluid containing the oocyte from the female patient's body using the needle guide. In such a procedure, a doctor who is performing the oocyte recovery procedure has both his/her hands occupied for holding the ultrasound probe and the attached needle guide. Meanwhile the Doctor also has to constantly observe an ultrasound monitor to be able to guide the needle tip in order to reach the correct spot in the ovary from where the oocyte(s) can be reached and aspirated.

The entire aspiration process is actually controlled by the doctor using a foot switch coupled with the aspiration pump. Upon pressing the foot switch, the follicle fluid along with the oocyte is sucked inside the needle and transferred to the test tube. At certain stage of the oocyte recovery procedure, it may be observed that the flow of the follicle fluid in the test tube may collapse and at this point the foot-switch has to be released by the doctor to neutralize the vacuum on the needle tip. Therefore, a constant monitoring of the follicle fluid falling in the test tube is required in the art. Specifically, it is required in the art to ensure that level of the follicle fluid doesn't exceed a pre-defined threshold level of a test tube in order to ensure, that the follicle fluid does not spill out of the test tube thereby preventing damage to already aspirated oocytes present in the test tube.

A constant watch on the level of the follicle fluid falling into the test tube is usually maintained by a nurse assisting the doctor. The nurse may orally communicate with the doctor, from time to time, regarding status of the level of the follicle fluid falling into the test tube. Since the status of the follicle fluid is relayed by the nurse, this may result in delay or error in communication. It is required in the art that the doctor gets uninterrupted and accurate information on the status of the level of the follicle fluid so that the doctor's vision is solely dedicated to screen of the ultrasound monitor at the time of the aspiration procedure.

In certain situations it may also happen that the passage within the needle or the tubing leading to the test tube gets blocked by a tissue fragment. At this stage the needle has to be extracted out of the patient and a higher level of vacuum may be applied to the set-up. The higher level of vacuum applied may help to remove the blockage in the pathway.

The drops of follicle fluid falling into a test tube directly indicate that there is no blockage in the needle or the tubing. In the situation where there is blockage, very few or no drops may be seen falling into the test tube. Since this droplet status is relayed by the nurse assisting doctor, delay in communication, error or loss in continuity may occur. It is therefore required to have a solution for the stated technical problems.

### SUMMARY

This summary is provided to introduce aspects related to detection of drop, hereinafter, also referred as droplet or droplets, of a follicle fluid falling into a test tube as well as level of follicle fluid falling in to a test tube during recovery of the follicle fluid from a patient's body. The inclusion of this summary is not intended to identify essential features of disclosure nor is it intended for use in determining or limiting the scope of the disclosure.

According to various aspects of present disclosure, an apparatus (with a drop detector and/or a level detector) and a method using such apparatus for detecting the droplets and level of the follicle fluid in the test tube is disclosed. The purpose of the drop detector is to detect first and subsequent drops of the follicle fluid and ensure the desired level of the follicle fluid in the test tube. Further, the detection of the first and the subsequent drops of the follicle fluid enable a doctor to ensure that needle is positioned into the patient's body at a correct location. Also, the detection of the drops ensures that there is no blockage in the path and the needle location is held in a correct position by the doctor.

Since the follicle fluid is collected in the test tube in a drop-wise manner, it is required to detect the first and the subsequent drops of the follicle fluid coming into the test tube. Upon detecting the first drop, an alert may be generated as an audio/visual signal as a part of this invention. The alert generated may indicate the user about the collection of the follicle fluid into the test tube.

The purpose of the apparatus is also to detect the level of the follicle fluid (having transparent and/or opaque characteristics). In one aspect, upon detecting the level of the fluid reaching the pre-defined level, an alert mechanism may generate an alert for indicating the doctor that the follicle fluid extracted in the test tube is reached to the pre-defined level. In one aspect, the alert generated may be an audio alert or a visual alert.

### BRIEF DESCRIPTION OF THE DRAWINGS

The detailed description is described with reference to the accompanying figures. In the figures.
Figure 1 is an Oocyte aspiration setup illustrating various components used in aspiration process for obtaining one or more oocyte from a female patient.
Figure 2 illustrates an apparatus comprising a Drop Detector Casing and a test tube in which the follicle drops are to be detected.
Figure 3 illustrates an arrangement of optical devices of the Drop Detector casing along with the test tube.
Figure 4 illustrates a functional block diagram of a Drop Detector.
Figure 5 illustrates a drop detect sensor for detecting droplets in the test tube in accordance with one embodiment of the present disclosure.
Figure 6 illustrates follicle fluid droplets of variable sizes and different colors that may be detected using the drop detector, in accordance with one embodiment of the present disclosure.
Figure 7 illustrates detail explanation of a detect zone (i.e., expected zone) and a sensing zone for detecting the droplets.
Figure 8 illustrates a method for detecting a drop of follicle fluid falling into a test tube, in accordance with an embodiment of the present disclosure.
Figure 9 illustrates an arrangement of casing comprising optical devices and other components for the level detection.
Figures 10(a) and 10(b) illustrates a functional block diagram of the level detection apparatus, in accordance with one embodiment of the present disclosure.

The figures depict embodiments of the present disclosure for purposes of illustration only. One skilled in the art will readily recognize from the following discussion that alternative embodiments of the apparatus and methods illustrated herein may be employed without departing from the principles of the disclosure described herein.

### DETAILED DESCRIPTION

Some embodiments of this disclosure, illustrating its features, will now be discussed in detail. The words "comprising," "having," "containing," and "including," and other forms thereof, are intended to be equivalent in meaning and be open ended in that an item or items following any one of these words is not meant to be an exhaustive listing of such item or items, or meant to be limited to only the listed item or items. It must also be noted that, the singular forms "a," "an," and "the" include plural references unless the context clearly dictates otherwise.

Various modifications to the embodiment of the invention as disclosed will be readily apparent to those skilled in the art and the generic principles herein may be applied to other embodiments. However, person skilled in the art will readily recognize that the present disclosure is not intended to be limited to the embodiments illustrated, but is to be accorded the widest scope consistent with the principles and features described herein.

### General working conditions and setup

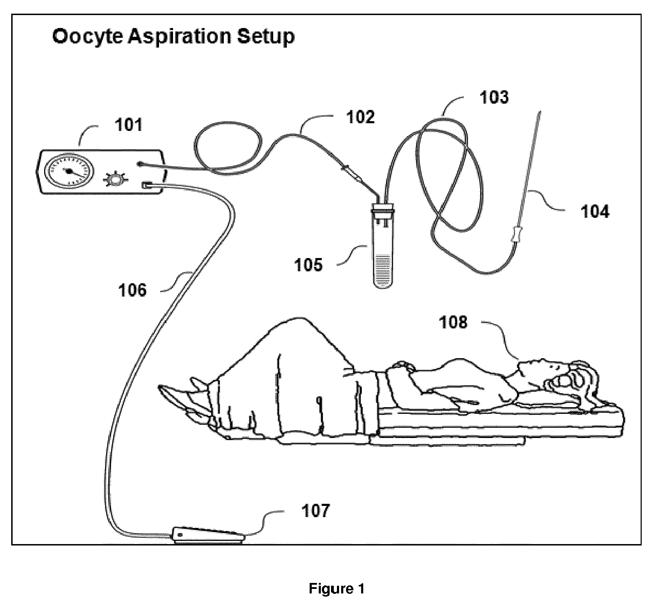

Referring to Figure 1, an Oocyte aspiration procedure is shown, in accordance with an embodiment of present disclosure. It may be seen from figure 1 that the female patient 108 is on an Operation Theatre (OT) table in a lithotomic position under anesthesia. Further, various articles for performing the oocyte aspiration are also illustrated, wherein said articles may comprise a vacuum generating unit 101, a suction tube 102, an inlet tube 103, a needle 104, a test tube 105, tube 106, and a foot switch/foot pedal 107.

The vacuum generating unit 101, also known as an aspirator, is a mains power operated device. The aspirator 101 is connected to the test tube 105 by the suction tube 102. The aspirator may be also connected to the foot switch 107, which is placed on the floor for the doctor to operate whenever he/she requires vacuum to be generated in the suction tube 102 which is further connected to the test tube 105.

The other tube i.e., the inlet tube 103 coming out from the test tube 105 is attached to the long needle 104. Further, the needle 104 may be used to insert into the patient till its tip reaches her ovaries, where the oocyte(s) are found suspended in the follicle fluid.

In the general practice, the test tube 105 is held in the hand by a nurse or any other person assisting the doctor during the oocyte aspiration procedure. The nurse is supposed to give a feedback or alarm the doctor orally about the follicle fluid status. Thus, during this oral communication there may be a chance of a miscalculation or misunderstanding or oversight amongst the nurse and the doctor. Hence, to overcome such situation one of the embodiment of the present disclosure provides a mechanism for automatically detecting the droplet falling into the test tube 105 or the level of the follicle fluid reaching a threshold and simultaneously notifying the doctor by means of an alarm (audio or visual).

### Drop Detection

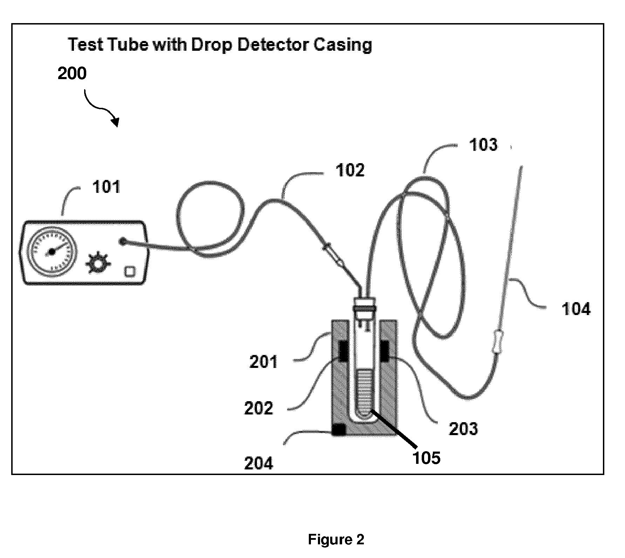

The first embodiment of the present invention relates to detection of droplets falling into a test tube and generating an audio or visual alarm. Referring to Figure 2, an apparatus 200 comprising an arrangement of a drop detector casing and the test tube is shown, in accordance with an embodiment of present disclosure. According to embodiments of present disclosure, as soon as a first drop of the droplets is detected into the test tube, the drop detector is enabled for generating an alert in form of an audio alarm or visual alarm. The test tube may be placed within a Drop Detector casing in order to enable electronic and optical components to work in unison for providing feedback to a doctor about the droplets falling into the test tube, in real time. Further, a detailed working of the drop detector is explained in detail in the following paragraphs.

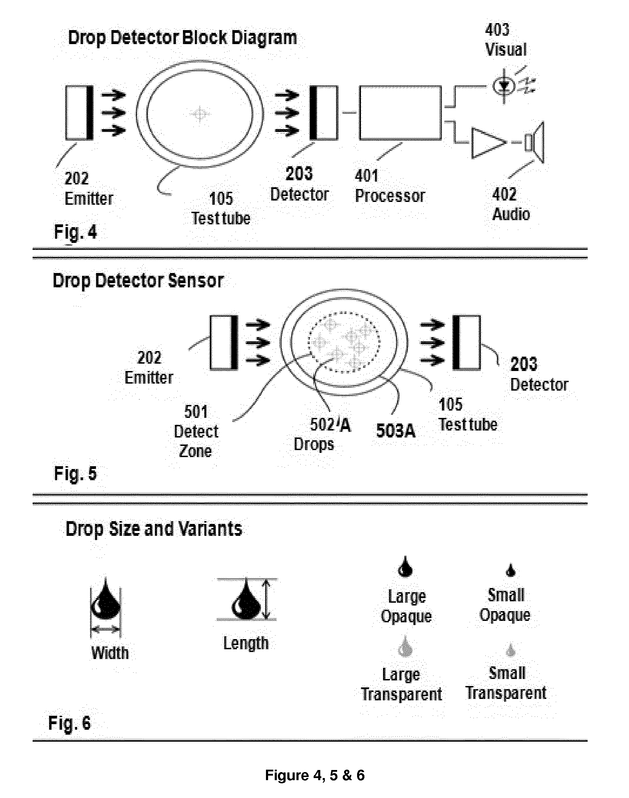

As illustrated in Figure 2, The Drop Detector Casing 201 will hold the test tube 105 in its designed cavity appropriately made to measure for the test tube 105. The test tube 105 is placed in the designed cavity in such a manner that it gets aligned with an optical devices i.e., an emitter 202 and detector 203 which are responsible for detecting the drops of the follicle fluid falling into the test tube 105. Further, a signal processing circuitry 204 along with other circuitries i.e., electrical and optical may reside inside the Drop Detector Casing 201. The signal processing circuitry 204 comprises a processor 401 (shown in figure 5), wherein the signal processing circuitry 204 may be further coupled with the test tube 105.

### Location of optics device

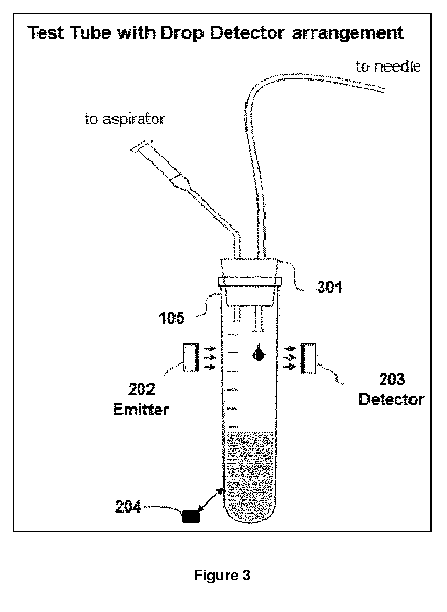

Figure 3 illustrates an arrangement of a pair of optical devices with the drop detector casing, in accordance with an embodiment of present disclosure. The optical devices comprise an emitter 202 and a detector 203. In one embodiment, the emitter 202 may be an infra-red (IR) light emitting diode and the detector 203 may be infra-red (IR) detector. The optical devices (202 and 203) are placed on opposite side of the test tube 105, wherein the opposite side refers to a position where the incoming fluid flow breaks into a drop and detaches from the end-point of the inlet tube 103 inside the test tube 105, before falling into the collected fluid in the test tube 105.

Figure 4 illustrates a functional block diagram of the drop detector, in accordance with an embodiment of present disclosure. The drop detector comprises the emitter 202, the detector 203, and a processor 401 of the signal processing circuitry 204, an audio indicator 402, and a visual indicator 403. The detail description of the drop detector is explained by referring to figure 5 as below.

Referring now to Figure 5, a drop detect sensor in shown, in accordance with an embodiment of present disclosure. The optical devices i.e., the emitter 202 and the detector 203 are placed on the opposite side of the test tube 105. The falling drop location cannot be ascertained to a defined spot. Thus, to detect the drops, a detect zone 501, also referred as an expected zone, may be defined within which every drop may fall. Further, the detect zone 501 is about 6 to 7 mm in diameter which is slightly smaller than the inner wall 503A of the test tube 105. The detect zone 501 is a result of the inlet tube 103 being at off-center of the cork with no fixed location for it to be placed onto. So each time someone places it on the test tube 105 its position can lie anywhere within the detect zone 501. Thus according to the invention, as long as the test tube 105 is positioned vertically and not shaken, the falling drop will always pass through the detect zone 501.

For detecting the first and the subsequent drops of the follicle fluid in the detect zone, the signal processing circuitry 204 (comprising the processor 401) coupled with the test tube 105, monitors the intensity of the beam of light received by the detector 203. Further, the signal processing circuitry 204 may compare the intensity of the beam of light with a pre-defined intensity. Based on the comparison, if a difference between the intensity and the pre-defined intensity is determined to be greater than a pre-defined threshold value, the signal processing circuitry 204 generates a valid electrical signal indicating the detection of the first drop of the follicle fluid falling into the test tube 105. Similarly, the subsequent drops of the follicle fluid may be detected by the signal processing circuitry 204 by comparing the intensity of the beam of light with the pre-defined intensity each time when the subsequent drops passes through the detect zone.

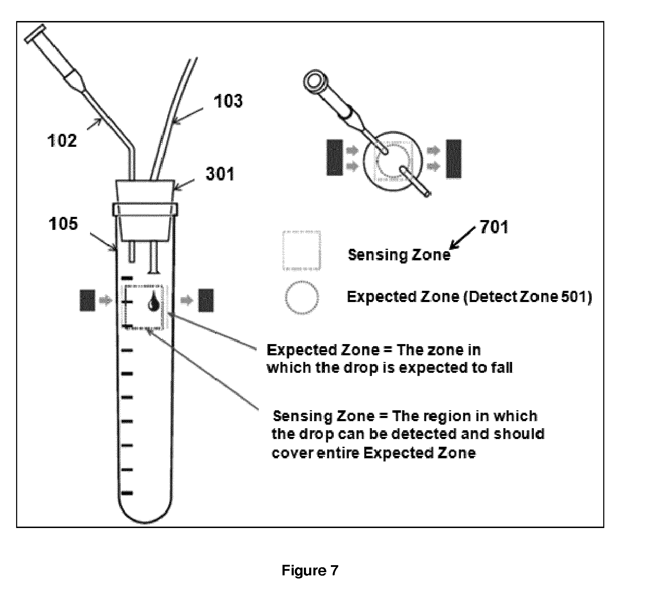

Further, the valid electrical signal may be used to initiate the audio indicator and visual indicator for generating an alarm. Thus, alarm may notify the doctor (or any user using the apparatus) about the timely retrieval of the follicle fluid in the test tube 105. According to embodiment of present disclosure, a sensing zone 701 as illustrated in figure 7 may be defined between the detect zone 501 and the inner wall 503A of the test tube 105. The purpose of defining the sensing zone 701 is to ensure that no droplet passes undetected while falling into the test tube 105. Since, the inlet tube 103 coming out from the needle 104 is not always placed at the center of a stopper, it may be required to define a zone for detecting the droplets falling into the test tube 105. When a nurse presses the stopper on the test tube 105 the position of falling droplets depends upon the stopper position. Since, the position of the stopper is never aligned there may be a deviation in the test tube where the droplets may be expected to fall. Thus, to overcome such scenario, an additional layer of zone may be defined i.e., the sensing zone 701. For example, assuming the inlet tube 103 is 3 mm off the axis which will give 6 mm circular zone as the expected zone i.e., the detect zone 501. Further, for providing safety margin of 0.5 mm at the radius, the sensing zone 701 may be defined which may be equal to 7 mm. These dimension may vary from case to case.

Referring now to Figure 6, a follicle fluid of different dimensions and color gradient is shown, in accordance with an embodiment of present disclosure. The follicle fluid retrieved from a patient may vary in its color and opacity, depending on several factors. The follicle fluid may be very light in color and almost transparent like light yellow colored water or in some instances may contain a large amount of blood, making it appear almost like the blood, dark and opaque. According to embodiments of present disclosure, the follicle fluid drop may vary in its width or diameter from 1.2 mm to 2.6 mm, whereas its length may vary from range of 2 mm to 4 mm. The optical and electronic circuitry combined together makes it possible to detect each and every drop falling into the test tube 105 irrespective of its size or optical characteristics. Thus, the fluid droplets of all sizes and colors may be detected without having to adjust or tune any part, mechanical, optical or electrical component of the drop detection device.

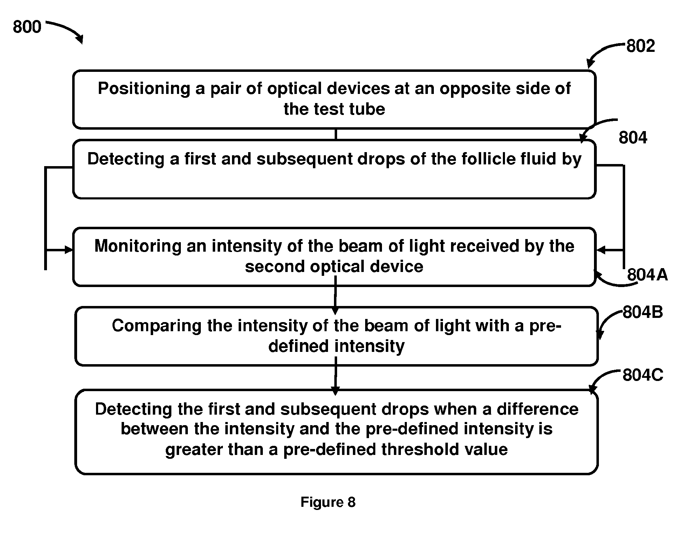

Referring now to Figure 8, method for detecting a drop of follicle fluid falling into a test tube is shown, in accordance with an embodiment of the present disclosure. The order in which the method 800 is described is not intended to be construed as a limitation, and any number of the described method blocks can be combined in any order to implement the method 800 or alternate methods. Additionally, individual blocks may be deleted from the method 800 without departing from the spirit and scope of the disclosure described herein. However, for ease of explanation, in the embodiments described below, the method 800 may be considered to be implemented in the above described apparatus 200.

At block 802, a pair of optical devices are positioned at opposite sides of the test tube 105. The test tube 105 has a cork fitted at open-end of the test tube 105, wherein the cork has a passage allowing an inlet tube 103, carrying the follicle fluid, to pass through the test tube 105. Further, the opposite side indicates a position on the test tube 105 where an incoming follicle fluid flow breaks into a drop and detaches from the inlet tube 103. Further, the pair of the optical devices comprises a first optical device and a second optical device. The first optical device emits a beam of light, through the test tube 105, towards the second optical device.

At block 804, the first and subsequent drops of the follicle fluid may be detected by performing the steps shown in the blocks 804A-804C.

At block 804A, an intensity of the beam of light received by the second optical device is monitored.

At block 804B, the intensity of the beam of light is compared with a pre-defined intensity.

At block 804C, the first and subsequent drops of the follicle fluid in the test tube 105 is detected when a difference between the intensity and the pre-defined intensity is determined to be greater than a pre-defined threshold value.

### Level Detection

Another embodiment of the invention is use of the apparatus for detecting level of follicle fluid containing oocyte extracted from a female patient's body in a test tube by employing a pair of optical devices. According to this embodiment, while extracting the follicle fluid, the apparatus may be configured to detect whether the level of the follicle fluid in the test tube reaches a pre-defined level. In order to detect the level of the follicle fluid, the test tube may be coupled within a casing that enables electronic and optical components to work in unison for providing feedback to a doctor about the level of the follicle fluid extracted into the test tube, in real time.

In one aspect, the electronic and optical components may include, but not limited to an array of photo emitters and an array of photo detectors. As soon as the level of the follicle fluid reaches the pre-defined level, an alert signal may be generated. In one aspect, the alert signal may be an audio alarm or a visual indicator that notifies the doctor that the follicle fluid falling into the test tube has reached the pre-defined level. The detail working of the level detection aspects of the apparatus are further explained in detail in subsequent paragraphs.

### Level detector Apparatus 500 & Location Of Optical Devices

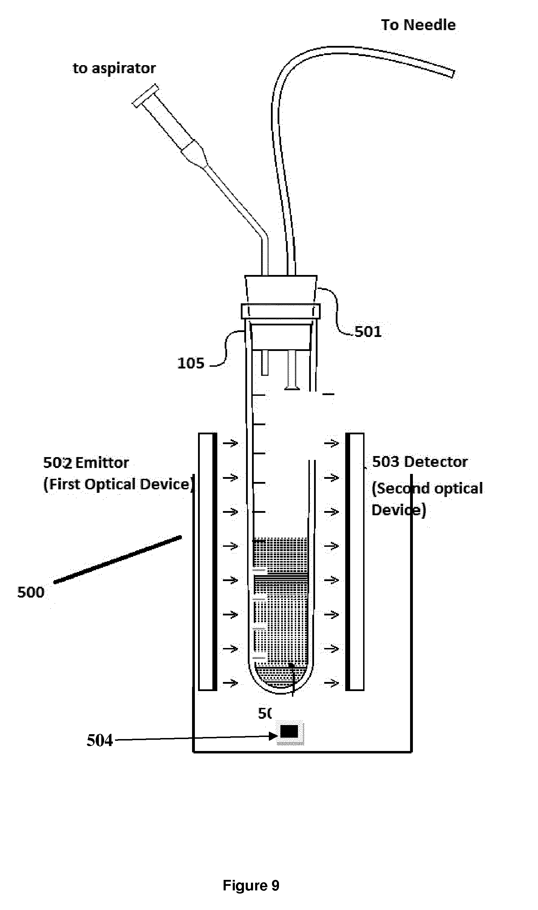

Referring to Figure 9, that illustrates arrangement of casing 501 comprising optical devices of the apparatus 500 along with the test tube 105, in accordance with an embodiment of present disclosure. As aforementioned, the optical devices may further comprise the first optical device 502 and the second optical device 503. In one embodiment, the first optical device 502 may be an array of infra-red (IR) light emitting diodes (LEDs) and the second optical device 503 may be an array of infra-red (IR) detectors. As shown in the Figure 9, the first optical device 502 and the second optical device 503 may be positioned, on the test tube 105, in a manner such that the second optical device 503 is aligned opposite to the first optical device 502.

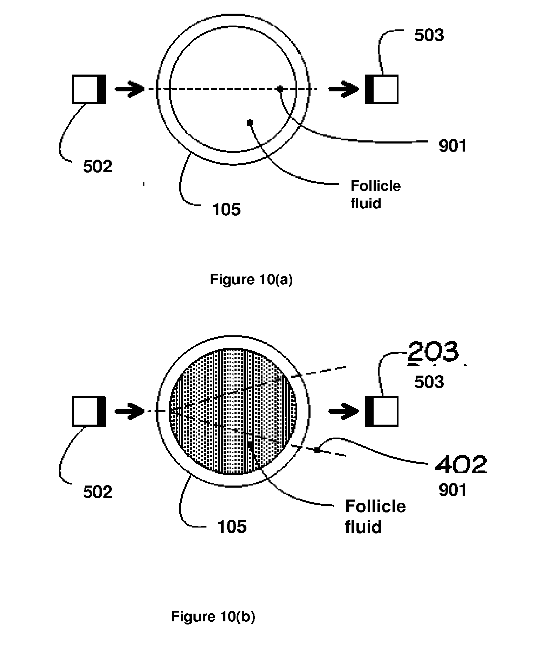

In one example, the test tube 105 may be coupled to the array of infra-red (IR) light emitting diodes, wherein each LED may be configured to emit a light beam through the test tube 105. It may be understood that from the Figure 9 that the test tube 105 may be divided into multiple sub-levels, wherein corresponding to each sub-level, an LED is deployed. Similarly, the test tube 105 may further be coupled to the array of IR detectors, wherein each IR detector is deployed corresponding to each LED of the test tube 105. Further, each IR detector may be configured to detect light beam emitted by the LED corresponding to the IR detector in order to detect whether the pre-defined threshold level of the follicle fluid in the test tube 105 has reached. In one aspect, whether the pre-defined threshold level has reached or not is determined by a signal processing circuitry 504 coupled with the casing 501. The signal processing circuitry 504 ensures the detection of the level of the fluid in the test tube 105. The functioning of the signal processing circuitry 504 is described in detail by referring the apparatus 500 illustrated in figure 10 as below.

### Detail Functioning of the Level detector Apparatus 500

Referring to Figure 10(a) and 10(b), a functional block diagram of the apparatus 500 is illustrated, in accordance with an embodiment of present disclosure. It may be understood that, the level detection of the follicle fluid is carried out by the optical devices, wherein the optical devices comprises a first optical device 502 and a second optical device 503, aligned opposite to the first optical device 502, on the test tube 105. In addition to the optics involved for detecting the level, a signal processing circuitry 504 (Not shown), coupled with the casing 501 (Not shown), further ensures the detection of the level of the fluid in the test tube 105. In one aspect, the first optical device 502, as aforementioned, may be the array of infra-red (IR) light emitting diodes (LEDs). Each infra-red LED may be deployed at a specific level in the test tube 105 for detecting the level of the follicle fluid in the test tube 105. For example, the array of infra-red (IR) LEDs may comprise a LED1, a LED2, a LED3, and a LED4 deployed at sub-levels of 5ml, 10ml, 15ml, and 20ml respectively of the test tube 105. It may be understood that, the light beam 901 emitted by the LED1, the LED2, the LED3, and the LED4 may be detected by the array of IR detectors Detector1, Detector2, Detector3 and Detector4 aligned on the opposite side of the LED1, the LED2, the LED3, and the LED4 respectively on the test tube 105. In one aspect, the Detector1, Detector2, Detector3 and Detector4 may be coupled with the test tube 105 corresponding to the LED1, the LED2, the LED3, and the LED4 respectively.

In order to detect the level, the signal processing circuitry 504, initially, monitors intensity of the beam received by the second optical device 503. After monitoring the intensity, the signal processing circuitry compares the intensity of the beam with a pre-defined intensity. Subsequently, the signal processing circuitry 504 detects the level of the follicle fluid in the test tube 105 when difference between the intensity and the pre-defined intensity is greater than a pre-defined threshold value. In one aspect, the level may indicate that the follicle fluid extracted in the test tube 105 has reached at a pre-defined level.

In one embodiment, the level of the follicle fluid may be detected by using the opacity property of the follicle fluid having opaque characteristics. In another embodiment, the level of the follicle fluid may be detected by using the refractive property of the follicle fluid having transparent characteristics.

In one example, in order to understand the working of the array of LEDs and the array of detectors in unison for the detection of the level of follicle fluid, consider the combination of the LED (502) and the Detector (503). As can be understood from the figure 10(a), the LED present in the first optical device 502 may constantly emit light beam 901 that may be detected by the Detector (503) located on the opposite side of LED. It may be understood that Detector detects the light beam 901 when there is no obstruction in the test tube 105 by any external means. This implies that the follicle fluid has not reached the pre-defined level (i.e. 5ml) marked in the test tube 105.

However, during the recovery procedure, when the follicle fluid is collected into the test tube 105, at a certain instance, the light beam 901 emitted by the first optical device 502 may be obstructed or refracted by the follicle fluid and hence does not allow the second optical device 503 to detect the light beam 901. It may be understood that the follicle fluid collected into the test tube 105 may have the opaque or the transparent characteristics. In one embodiment, when the follicle fluid is having the opaque characteristics, then there will be obstruction created by the follicle fluid (e.g. assuming the follicle present in the follicle fluid is dark in color) for the passage of the light beam 901 emitted by the LED to the Detector 503. This in turn, enables the second optical device 503 to ensure that the follicle fluid has reached the pre-defined level for example, 5 ml in this case.

In another embodiment, when the follicle fluid is having the transparent characteristics, then there will be no obstruction created by the follicle fluid. Since the follicle fluid is having transparent characteristics, the light beam 901 emitted by the LED may pass through the follicle fluid and thereby causing refraction of the light beam 901 due to the presence of the follicle fluid in the test tube 105. Since, the light beam 901 is refracted, the Detector 503 corresponding to the LED may not be able to detect the light beam 901. This in turn, enables the detector 504 to ensure that the follicle fluid has reached the pre-defined level.

Similarly, using the combinations of other LEDs and the corresponding IR detectors, the apparatus 500 may be enabled to ensure that the follicle fluid has reached the multiple sub-levels (e.g. 10ml, and 15ml) of the test tube 105. In one aspect, the apparatus 501, via the signal processing circuitry 504, may further facilitate the doctor to view on the ultrasound monitor, the gradual filling of the test tube 105 with the follicle fluid, as-and-when the follicle fluid reaches the multiple sub-levels, and finally the pre-defined threshold level is reached.

Once the follicle fluid reaches the pre-defined level, an alert mechanism enables the doctor to stop the extraction and/or retrieval of oocytes from the female patient's body and thereby conclude the collection of the follicle fluid in the test tube 105. In one aspect, the alert may be generated as an audio alert or a visual alert. In one aspect, the audio indicator may be an audio signal such as buzzer. The alert mechanism facilitates to prevent spilling of the follicle fluid from the test tube 105 during the extraction of the oocyte from the female patient's body, once the pre-defined level is reached.

Although implementations for methods for detecting level of follicle fluid have been described in language specific to structural features and/or methods, it is to be understood that the implementations and/or embodiments are not necessarily limited to the specific features or methods described.

## Claims

1. An apparatus for detecting a drop of follicle fluid falling into a test tube, the apparatus comprising:
a test tube (105) for storing follicle fluid, wherein the test tube 105 has a cork fitted at open-end of the test tube (105), and wherein the cork has a passage allowing an inlet tube 103, carrying the follicle fluid, to pass through the test tube (105);
a pair of optical devices positioned at an opposite side of the test tube (105), wherein the opposite side indicates a position on the test tube (105) where an incoming follicle fluid flow breaks into a drop and detaches from the inlet tube (103), and wherein the pair of the optical devices comprises a first optical device and a second optical device, and wherein the first optical device emits a beam of light, through the test tube (105), towards the second optical device; and
a signal processing circuitry (204), coupled with the test tube (105), detects first and subsequent drops of the follicle fluid by
monitoring an intensity of the beam of light received by the second optical device,
comparing the intensity of the beam of light with a pre-defined intensity, and
detecting the first and subsequent drops of the follicle fluid falling in the test tube (105) when a difference between the intensity and the pre-defined intensity is greater than a pre-defined threshold value.

2. The apparatus of claim 1 further comprising an alert mechanism for generating an audio or visual alert for indicating an operator that the first and subsequent drops of the follicle fluid are falling into the test tube (105).

3. The apparatus of claim 1, wherein the difference is based on opacity or transparency properties of the follicle fluid.

4. A method for detecting a drop of follicle fluid falling into a test tube, the method comprising:
positioning a pair of optical devices at an opposite side of the test tube (105), wherein the test tube (105) has a cork fitted at open-end of the test tube (105), and wherein the cork has a passage allowing an inlet tube (103), carrying the follicle fluid, to pass through the test tube (105), and wherein the opposite side indicates a position on the test tube (105) where an incoming follicle fluid flow breaks into a drop and detaches from the inlet tube (103), and wherein the pair of the optical devices comprises a first optical device and a second optical device, and wherein the first optical device emits a beam of light, through the test tube (105), towards the second optical device; and
detecting, by a signal processing circuitry (204) coupled with the test tube (105), first and subsequent drops of the follicle fluid by
monitoring an intensity of the beam of light received by the second optical device, comparing the intensity of the beam of light with a pre-defined intensity, and
detecting the fall of first and subsequent drops of the follicle fluid in the test tube (105) when a difference between the intensity and the pre-defined intensity is greater than a pre-defined threshold value.

5. The method of claim 4, further comprising generating an alert, by an audio or visual alert mechanism, for indicating an operator that the first and subsequent drops of the follicle fluid are falling into the test tube (105).

6. The method of claim 4, wherein the difference is based on opacity or transparency properties of the follicle fluid.

7. A method for detecting level of follicle fluid in a test tube 105, the method comprising:
positioning one or more first optical device (502) and one or more second optical device (503), on a test tube (105), in a manner such that the second optical device (503) is aligned opposite to the corresponding first optical device (502), wherein the first optical device (502) emits beam, through the test tube (105), towards the second optical device (503);
extracting follicle fluid in the test tube (105) by generating vacuum pressure using an aspirator;
monitoring, via a signal processing circuitry (504), intensity of the beam received by the second optical device (503);
comparing, via a signal processing circuitry (504), the intensity of the beam with a pre-defined intensity; and
detecting, via a signal processing circuitry (504), the level of the follicle fluid in the test tube 105 when difference between the intensity and the pre-defined intensity is greater than a pre-defined threshold value, wherein the level indicates that the follicle fluid extracted in the test tube (105) is reached to a pre-defined level.

8. The method of claim 7 further generates an audio or visual alert for indicating an operator that the follicle fluid extracted in the test tube (105) is reached to the pre-defined level.

9. The method of claim 7, wherein the difference is based on the opacity or transparency properties of the follicle fluid.

10. An apparatus (500) for detecting level of follicle fluid in a test tube 105, the apparatus comprising:
a test tube (105) for storing follicle fluid;
a casing (501) coupled with the test tube (105), wherein the casing (501) comprises a first optical device (502) and a second optical device (503), and wherein the first optical device (502) and the second optical device (503) are positioned, on the test tube (105), in a manner such that the second optical device (503) is aligned opposite to the first optical device (503), and wherein the first optical device (502) emits beam, through the test tube (105), towards the second optical device (503); and
a signal processing circuitry (504), coupled with the casing (501), facilitates to detect the level of the follicle fluid by
monitoring intensity of the beam received by the second optical device (503),
comparing the intensity of the beam with a pre-defined intensity, and
detecting the level of the follicle fluid in the test tube (105) when difference between the intensity and the pre-defined intensity is greater than a pre-defined threshold value, wherein the level indicates that the follicle fluid extracted in the test tube (105) is reached to a pre-defined level.

11. The apparatus of claim 10 further an alert mechanism for generating an audio or visual alert for indicating an operator that the follicle fluid extracted in the test tube (105) is reached to the pre-defined level.

12. The apparatus of claim 10 wherein the difference is based on opacity or transparency properties of the follicle fluid.
